# EUROPÄISCHE PATENTSCHRIFT

(11) **EP 1 709 914 B1**
(45) Veröffentlichungstag und Bekanntmachung des Hinweises auf die Patenterteilung: **23.07.2008**
(21) Anmeldenummer: 06008523.0
(22) Anmeldetag: 26.02.2003
(51) Int. Cl.: A61B 17/15, A61B 17/02

(54) **Bänderspannvorrichtung mit Schnittlehre**
Ligament tensioning device with cutting jig
Dispositif de tensionnement de ligaments comportant un modèle de coupe

(30) Priorität: 08.04.2002 DE 10215358
(43) Veröffentlichungstag der Anmeldung: 11.10.2006
(62) Teilanmeldung aus: 03745688.6
(73) Patentinhaber: Mathys Medizinaltechnik AG, 2544 Bettlach (CH)
(72) Erfinder: Delfosse, Daniel, Dr., 3303 Jegenstorf (CH); Supper, Walter, 2540 Grenchen (CH); Grunder, Beat, 3076 Worb (CH)
(74) Vertreter: Körfer, Thomas

(56) Entgegenhaltungen:
- EP-A- 0 809 969
- WO-A-00/78225
- WO-A-01/85038
- US-A- 4 566 448

## Beschreibung

Die Erfindung betrifft eine Bänderspannvorrichtung mit einer Schnittlehre für Gelenke des menschlichen oder tierischen Körpers sowie ein Verfahren zur Osteotomie dieser Gelenke unter Verwendung der erfindungsgemäßen Bänderspannvorrichtung mit Schnittlehre.

Aus der WO 00/78225 A1 ist eine Bänderspannvorrichtung für nicht-kugelige Gelenke bekannt. Die darin beschriebene Vorrichtung zum Spannen von Bändern an nicht-kugeligen Gelenken am menschlichen oder tierischen Körper umfaßt einen prismatischen, zylindrischen oder plattenförmigen Grundkörper mit einer rechten Pratze und einer linken Pratze, welche erste Auflageflächen in einer Ebene aufweisen und damit parallel auf die gelenkseitige Oberfläche eines ersten an ein nicht-kugeliges Gelenk angrenzenden Knochens zur Anlage bringbar sind, sowie einen rechten Handgriff und einen linken Handgriff, einen rechten Spannhebel und einen linken Spannhebel mit zweiten Auflageflächen, welche parallel zu den ersten Auflageflächen angeordnet sind, wobei zwischen den jeweiligen Auflageflächen des rechten Spannhebels und der rechten Pratze eine Spannweite Y und zwischen den jeweiligen Auflageflächen des linken Spannhebels und der linken Pratze dieselbe oder eine andere Spannweite X einstellbar ist. Die zweiten Auflagefläche sind auf die gelenkseitige Oberfläche eines zweiten an das Gelenk angrenzenden Knochens zur Anlage bringbar. Weiterhin umfaßt die Vorrichtung einen rechten Bedienungshebel und einen linken Bedienungshebel, welche gleichzeitig mit dem Halten der Vorrichtung mit je einer Hand am entsprechenden Handgriff einzeln mit der jeweils selben Hand betätigbar sind und eine rechte Parallelverschiebevorrichtung und eine linke Parallelverschiebevorrichtung, welche je durch den entsprechenden Bedienungshebel antreibbar sind und so mit je einem Spannhebel verbunden sind, daß bei einer Bewegung der Bedienungshebel die Spannweiten X bzw. Y unabhängig voneinander einstellbar sind. Die Parallelverschiebevorrichtungen sind als Viergelenk-Hebelgetriebe ausgebildet.

Nachteilig an der aus der WO 00/78225 A1 bekannten Bänderspannvorrichtung ist insbesondere, daß die Anbringung von Schnittebenen an einem erkrankten Gelenk zur Einbringung einer Prothese weitere Werkzeuge erfordert, welche unabhängig von der Spannvorrichtung an das Gelenk angesetzt werden und dadurch keine genaue Positionierung und Ausrichtung sowie keine reproduzierbare, genaue Schnittführung erlauben.

WO 01/85038 weist eine Bänderspannvorrichtung mit den Merkmalen der Oberbegriffs des Anspruchs 1 auf.

Der Erfindung liegt demnach die Aufgabe zugrunde, eine Bänderspannvorrichtung zu schaffen, um die Kapsel-Bandstrukturen eines prothetisch zu versorgenden Gelenkes mit einer parallelen Spreizbewegung anzuspannen und dabei eine voreinstellbare, nachjustierbare und nachkontrollierbare Schnittführung bei der Vorbereitung und Durchführung der für die prothetische Versorgung eines Gelenkes benötigten Anschnitte zu ermöglichen.

Die Aufgabe wird hinsichtlich der Bänderspannvorrichtung durch die Merkmale des Anspruchs 1 gelöst.

Weitere vorteilhafte Ausgestaltungen der Erfindung sind in den Unteransprüchen gekennzeichnet.

Die Schnittlehre weist Fortsätze mit U-förmigen Schlitzen auf, welche in die Halterungen der Bänderspannvorrichtung aufsteckbar und rastend mittels eines Arretierungselements fixierbar sind.

Weiterhin ist von Vorteil, daß über zwei korrespondierende Skalen, welche die jeweilige Stellung der Bänderspannvorrichtung definieren, die Weite des Kniegelenksspalts bzw. die Dicke des einzubringenden Implantats voreinstellbar und zu jedem Zeitpunkt der Operation kontrollierbar ist.

Die Schnittlehre weist dabei vorteilhafterweise eine Sägeführung auf, welche die Führung der Knochensäge bei sehr geringen Versätzen und hoher Schnittgenauigkeit ermöglicht. Weiterhin ist vorzugsweise eine Zylinderführung vorgesehen, welche die Anbringung weiterer Operationsinstrumente an der Schnittlehre ermöglicht.

In einer weiteren vorteilhaften Ausführungsform kann die Bänderspannvorrichtung auch als zweiseitige Bänderspannvorrichtung ausgebildet sein, welche die gleichzeitige Versorgung des medialen und des lateralen Gelenkteils ermöglicht.

Die Erfindung wird im folgenden anhand teilweise schematischer Darstellungen für die Vorbereitung der prothetischen Versorgung eines menschlichen Kniegelenks näher erläutert.

Es zeigen:
- Fig. 1A: eine schematische, perspektivische Ansicht einer Bänderspannvorrichtung mit einer erfindungsgemäß ausgestalteten Schnittlehre,
- Fig. 1B: eine vergrößerte Darstellung der in Fig. 1A dargestellten Schnittlehre,
- Fig. 2A-J: schematische, perspektivische Darstellungen einer distalen Femurosteotomie unter Verwendung der erfindungsgemäßen Schnittlehre,
- Fig. 3A-F: schematische, perspektivische Darstellungen einer dorsalen Femurosteotomie unter Verwendung der erfindungsgemäßen Schnittlehre, und
- Fig. 4A-J: schematische, perspektivische Darstellungen femoraler Schrägschnitte unter Verwendung der erfindungsgemäßen Schnittlehre.

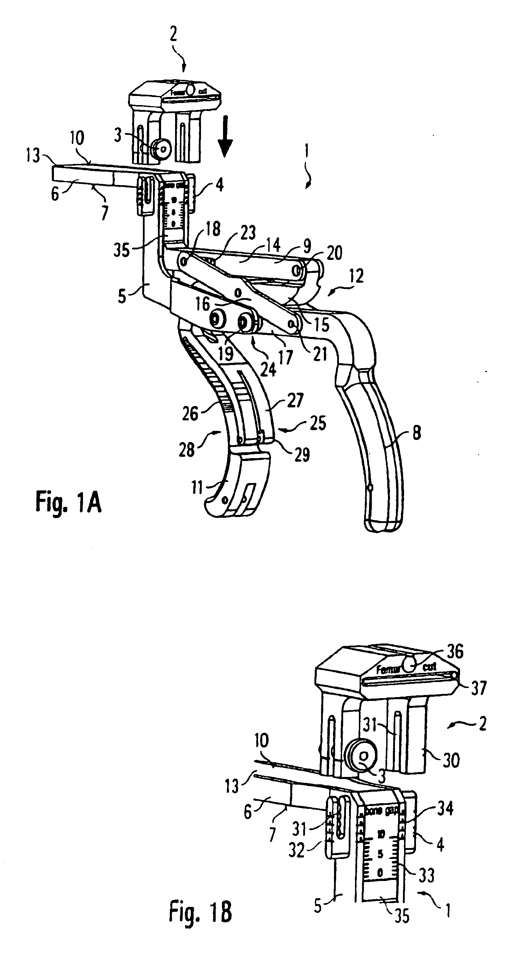

Fig. 1A zeigt in einer schematischen, perspektivischen Gesamtdarstellung eine Bänderspannvorrichtung 1, auf welche eine Schnittlehre 2 aufsteckbar ist. Die Schnittlehre 2 ist mittels einer Arretiervorrichtung 3 auf Halterungen 4 der Bänderspannvorrichtung 1 aufsteckbar und an diesen arretierbar.

Die Bänderspannvorrichtung 1 umfaßt einen Grundkörper 5, welcher zur sicheren Einleitung der Spreizkraft in die Tibia über eine erste Pratze 6 mit einer in Bezug auf den Kniegelenksspalt distalen Auflagefläche 7 verfügt, welche im Fall des Kniegelenks auf dem Femur aufliegt. Der ersten Pratze 6 gegenüberliegend ist entsprechend am Grundkörper 5 ein Handgriff 8 angebracht, welcher ein einhändiges Halten und Spannen der Bänderspannvorrichtung 1 ermöglicht. Ebenfalls entsprechend zur Anordnung der ersten Pratze 6 und oberhalb dieser liegend umfaßt die Bänderspannvorrichtung 1 einen Spannhebel 9, welcher sich mit seiner auf einer zweiten Pratze 13 ausgebildeten proximalen Auflagefläche 10 auf dem gegenüberliegenden Anteil des zu behandelnden Gelenkes, im Fall des Kniegelenks der Tibia, abstützt. Die Spreizwirkung wird durch Betätigen des Handgriffs 8 zusammen mit einem Bedienungshebel 11 jeweils wahlweise für einen medialen oder lateralen Gelenkanteil erzeugt.

Eine Parallelverschiebevorrichtung 12 gestattet bezüglich der Auflageflächen 7 und 10 eine Parallelverschiebung der zweiten Pratze 13 mit der Auflagefläche 10 gegenüber der ersten Pratze 6 mit der Auflagefläche 7. Die zweite Pratze 13 steht dabei in Wirkverbindung mit dem Spannhebel 9. Die Parallelverschiebevorrichtung 12 ist als Viergelenk in Form sich kreuzender Stäbe ausgeführt und umfaßt vier Hebel 14, 15, 16, 17, wobei ein spannhebelseitiger Hebel 14 und ein grundkörperseitiger Hebel 17 parallel angeordnet sind, während sich die Hebel 15 und 16 kreuzen. Die vier Hebel 14, 15, 16, 17 sind mittels fünf Achsen 18, 19, 20, 21, 22 miteinander verbunden. Zwei der Achsen 18, 19 sind in den parallelen Hebeln 14, 17 in parallel zu den Auflageflächen 7, 10 verlaufenden Langlöchern 23, 24 verschiebbar gelagert. Dieser Ausgestaltung der Parallelverschiebevorrichtung 12 gestattet, daß der spannhebelseitige Hebel 14 und der grundkörperseitige Hebel 17 parallel zueinander bzw. auseinander bewegbar sind. Die Längen der Hebel 14, 15, 16, 17 sind so gewählt, daß bei einer beliebigen Spannweite X zwischen der Auflagefläche 7 an der ersten Pratze 6 und der Auflagefläche 10 an der zweiten Pratze 13, welche z. B. zwischen 5 mm und 40 mm liegen kann, ein konstantes Umsetzungsverhältnis von 1:1 zwischen der manuell an dem Handgriff 8 und dem Bedienungshebel 11 aufgebrachten Spannkraft und der auf die an das Gelenk angrenzenden Knochen ausgeübten Distraktionskraft herrscht.

Die Größe der Spreizkraft ist an einer Kraftanzeige 25 mit einer Skala 26 und einem beweglichen Anzeigehebel 27 ablesbar. Der Anzeigehebel 27 wird durch die longitudinale Biegung des durch eine manuell aufgebrachte Spannkraft biegbaren Bedienungshebelteils 28 gegenüber dem anderen gabelartig angeordneten und nicht durch diese Spannkraft beaufschlagten Anzeigehebel 27 bewegt. Werden mittels der Spannkraft der Anzeigehebel 27 und das Bedienungshebelteil 28 relativ zueinander bewegt, dreht sich der Anzeigehebel 27 um eine Drehachse 29, wodurch auf der Skala 26 durch den Anzeigehebel 27 die manuell aufgebrachte Spannkraft angezeigt wird.

Weiterhin kann zwischen dem Handgriff 8 und dem Bedienungshebel 11 eine in Fig. 1A nicht weiter dargestellte Arretierungsvorrichtung vorgesehen sein, welche die Arretierung der Bänderspannvorrichtung 1 in einer bestimmten Position ermöglicht.

Die erste Pratze 6 und die zweite Pratze 13 sind im Ausführungsbeispiel als äußere Pratze 6 und innere Pratze 13 ausgebildet, welche im entspannten Zustand der Bänderspannvorrichtung 1 in einer Ebene liegen und eine geschlossene distale und proximale Auflagefläche bilden. Dies erleichtert das Einführen der Bänderspreizvorrichtung in das zu behandelnde Gelenk. Bei Betätigung der Bänderspannvorrichtung 1 wird die innere Pratze 13 gegenüber der äußeren Pratze 6 parallel verschoben.

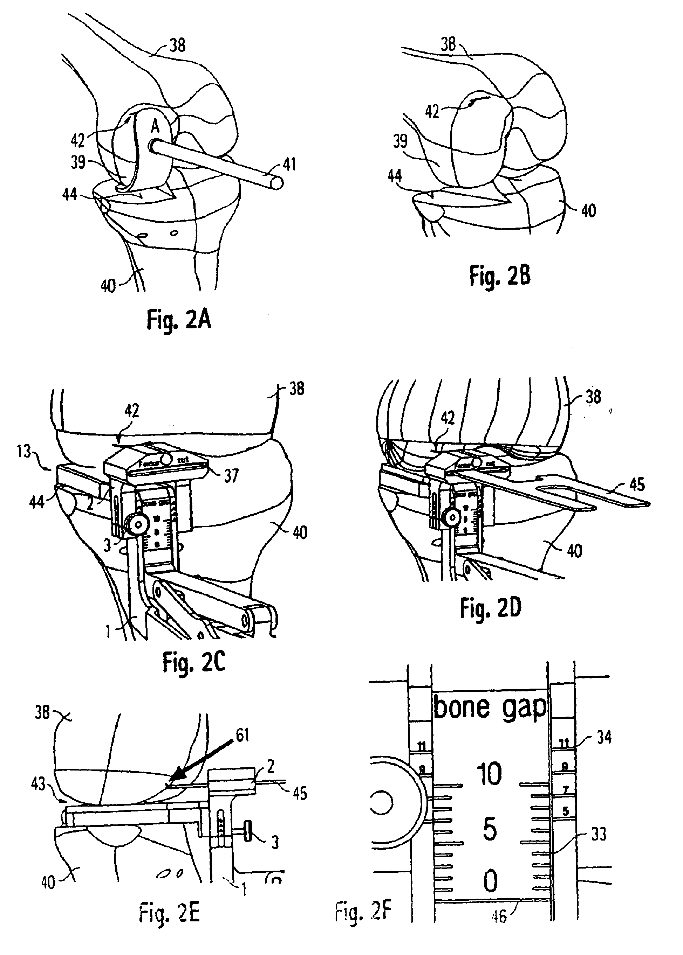

Der Grundkörper 5 der Bänderspannvorrichtung 1 weist eine erste Skala 34 auf, welche mit einer zweiten Skala 34 auf einem den Hebel 14 mit der zweiten Pratze 13 verbindenden Bauteil 35 korrespondiert. Die Skalen 33 und 34 dienen der Voreinstellung sowie der Kontrolle der Weite des Kniegelenksspaltes vor und nach den die Implantierung vorbereitenden Osteotomien. Die genaue Funktion der Skalen 33 und 34 ist in Fig. 2F sowie in der dazugehörigen Beschreibung näher erläutert.

Die Bänderspannvorrichtung 1 kann auch als doppelseitige Bänderspannvorrichtung 1 mit zwei zueinander parallel wirkenden Bänderspannvorrichtungen 1, welche in beliebiger Weise beispielsweise im Bereich des Grundkörpers 5 miteinander verbunden sein können und eine gleichzeitige Versorgung des medialen und des lateralen Gelenkanteils ermöglichen, ausgebildet sein. Dabei sind dann ebenfalls ein oder zwei auf die Bänderspannvorrichtung 1 aufsetzbare Schnittlehren 2 vorzusehen.

Fig. 1B zeigt in einer vergrößerten Ansicht den Bereich der Bänderspannvorrichtung 1, an welchem die Schnittlehre 2 montiert wird. Die Schnittlehre 2 weist dabei zwei Fortsätze 30 auf, welche U-förmig ausgebildet sind, wobei Schlitze 31 gebildet werden, welche mit den Halterungen 4 am Grundkörper 5 der Bänderspannvorrichtung 1 beim Aufsetzen der Schnittlehre 2 in Eingriff kommen. Eine der Halterungen 4 ist dabei ebenfalls U-förmig ausgebildet, wobei der hierdurch ausgebildete Schlitz 31 Rasten 32 aufweist, welche mit dem Arretierungselement 3 so in Eingriff kommen, daß die Schnittlehre 2 in z. B. äquidistanten Schritten von beispielsweise 2 mm auf dem Grundkörper 5 der Bänderspannvorrichtung 1 verschiebbar arretierbar ist.

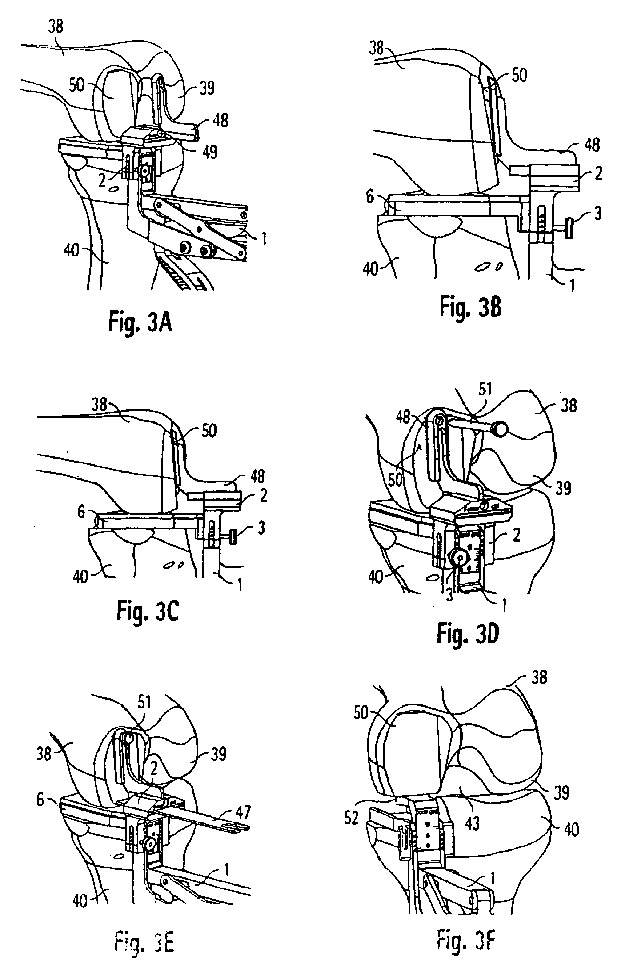

Die Schnittlehre 2 weist weiterhin eine Zylinderführung 36 auf, in welche weitere Instrumente zur Durchführung oder Positionierung der Osteotomien eingeführt werden können. Dies kann beispielsweise, wie in Fig. 3A bis 3E ersichtlich, eine Richtlehre 48 sein, welche eine feste Positionierung der Schnittlehre 2 unter einem festgelegten Winkel ermöglicht.

Zur Führung einer Tastlehre oder der für die Osteotomien zu verwendenden Knochensäge ist eine Sägeführung 37 vorgesehen, welche vorzugsweise rechtwinklig in der Schnittlehre 2 ausgebildet ist. Die Sägeführung 37 sorgt für eine abweichungsfreie Führung der Säge, wodurch bei der Resektion der betreffenden Knochenpartien eine hohe Genauigkeit bei geringen Versätze erzielt wird.

Die folgenden Figuren 2A bis 2J bis 4A bis 4J zeigen die Arbeitsschritte, welche benötigt werden, um den Femur 38 im Bereich des Kniegelenks auf die Implantierung eines einen beispielsweise arthrotisch zerstörten Femurkondylus 39 ersetzenden Implantats vorzubereiten. Die vorbereitenden Maßnahmen an der Tibia 40 können dabei mittels bereits bekannter Resektionsmethoden erfolgen.

Die Fig. 2A bis 2J zeigen die vorbereitenden Arbeiten für die distale Femurosteotomie. Zu diesem Zweck wird zunächst, wie nicht weiter erläutert, die Tibia 40 entsprechend vorbereitet, wie in Fig. 2A bereits ersichtlich. Ebenfalls aus Fig. 2A ist ersichtlich, wie mittels einer Femur-Größenlehre 41 die Größe des zu resezierenden Femur 39 bestimmt wird. Dem Operateur stehen dabei mehrere z. B. fünf Größen zur Verfügung, um die Größe des Femur 38 korrekt zu bestimmen. Von Interesse für die distale Femurosteotomie ist dabei zunächst die in Fig. 2A mit 42 gekennzeichnete Markierung, welche auf dem Femur 38 angebracht wird. Dabei wird das Bein zunächst in 90°-Stellung gebracht und nach der Positionierung der Femur-Größenlehre 41 am ventralen Ende mit Hilfe eines Elektrokauter eine Markierung 42 am Femur 38 gemacht. Die Position dieser Markierung ist in etwa die Grenze zwischen dem femurotibialen und dem femuropatellaren Teil des Femur 38. Nach Entfernen der Femur-Größenlehre 41 ist die angebrachte Markierung 42, wie in Fig. 2B ersichtlich, auf dem Femur 38 sichtbar.

Im nächsten Schritt wird, wie in Fig. 2C dargestellt, die Schnittlehre 2 an der Bänderspannvorrichtung 1 vormontiert, wobei die Schnittlehre 2 auf die Bänderspannvorrichtung 1, wie in Fig. 1A und 1B beschrieben, aufgesteckt wird. Mit Hilfe des Arretierungselementes 3 kann, wie bereits weiter oben erwähnt, die Dicke des später einzusetzenden Inlay-Implantats zwischen 5 und 11 mm in Abstufungen von 2 mm eingestellt werden.

Danach wird bei gestrecktem Bein die Bänderspannvorrichtung 1 mit montierter Schnittlehre 2 in den Gelenkspalt 43 eingeführt. Dabei liegt die distale Auflagefläche 7 der ersten Pratze 6 der Bänderspannvorrichtung 1 auf der bereits vorbereiteten Tibia-Schnittfläche 44 auf.

Nun wird die Bänderspannvorrichtung 1 mit der gewünschten Kraft aufgespreizt. Eine Tastlehre 45 wird durch die Sägeführung 37 der Schnittlehre 2 geführt, bis die Tastlehre 45 am Femur 38 anliegt. Nun wird kontrolliert, ob die Markierung 42 am Femur 38 in der Flucht der Tastlehre 45 liegt. Dies ist aus den Fig. 2D und 2E ersichtlich. Der Pfeil 61 in Fig. 2E verdeutlicht, daß die Tastlehre 45 nun in der gewünschten Position an der Markierung 42 anliegt bzw., daß der Schnittverlauf für die distale Femurosteotomie korrekt eingestellt ist. Dies kann mittels einer gezielten Bewegung des Unterschenkels optimal eingestellt werden.

Nach dem Aufspreizen der Bänderspannvorrichtung 1 kann bereits vor der Resektion die Dicke des zu reseszierden distalen Femurkondylus 39 anhand des Maßstabes auf der Skala 34 der Bänderspannvorrichtung 1 abgelesen werden. Die Distanz ermittelt sich aus der Differenz von der Null-Linie 46 bis zur gewählten Dicke des später einzusetzenden Implantats. In Fig. 2F beträgt die zu resezierende Kondylendicke bei Auswahl eines 5 mm Inlay-Implantats 7 mm. Beträgt der gemessene Wert weniger als 5 mm, muß die Schnittlehre 2 entsprechend auf eine dickere Implantatstärke eingestellt werden, z. B. auf 7 oder 9 mm. Ist die gemessene Differenz größer als 8 mm, z. B. bei eingestellten Kondylenhöhe von 5 mm, so muß eine Nachresektion an der Tibia 40 vorgenommen werden.

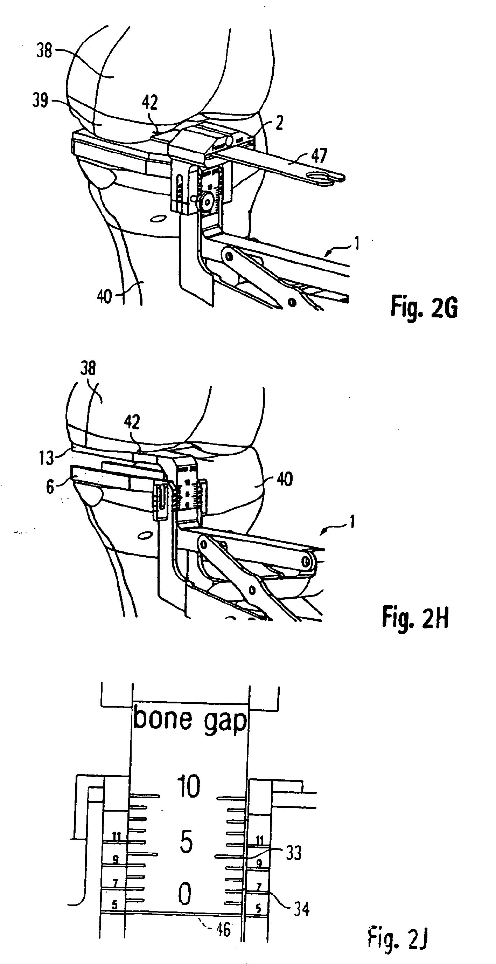

Nach den vorbereitenden Arbeiten wird die distale Femurosteotomie mittels einer durch die Sägeführung 37 der Schnittlehre 2 geführten Säge 47 durchgeführt. Dies ist in Fig. 2G dargestellt.

Nach Ausführung der distalen Femurosteotomie wird die Bänderspannvorrichtung 1 entspannt und aus dem Kniegelenksspalt 43 entfernt. Die Schnittlehre 2 wird von der Bänderspannvorrichtung 1 demontiert. Danach wird die Bänderspannvorrichtung 1 wiederum in den Gelenkspalt 43 eingebracht. Nun wird durch Aufspreizen der Bänderspannvorrichtung 1 die Weite des Gelenkspalts 43 kontrolliert. Die Null-Linie 46 muß mit der gewählten Implantatdicke übereinstimmen, wie in Fig. 2H und Fig. 2J dargestellt.

Im Beispiel ist die Übereinstimmung mit der vorgewählten Implantatdicke von 5 mm ersichtlich, da die Null-Linie 46 der Skala 33 jetzt mit der 5mm-Linie der Skala 34 zusammenfällt, wie in Fig. 2J dargestellt. Bei Abweichungen, welche mehr als einen Millimeter betragen, kann mittels einer Nachresektion an der Tibia 40 oder an der distalen Femurkondüle eine Korrektur vorgenommen werden.

Die Fig. 3A bis 3F zeigen den nun folgenden Schritt der dorsalen Femurosteotomie. Zu diesem Zweck wird das Bein zunächst wieder in eine 90° Beugestellung gebracht.

An der Bänderspannvorrichtung 1 wird wiederum die Schnittlehre 2 vormontiert. Dabei ist die Schnittlehre 2 auf die gleiche Inlay-Implantatdicke wie bei der vorangegangene distalen Femurosteotomie eingestellt. Die vormontierte Bänderspannvorrichtung 1 wird nun in den Gelenkspalt 43 eingebracht. Danach wird die Bänderspannvorrichtung 1 unter der gewünschten Kraft aufgespreizt.

Nun wird, wie in Fig. 3A dargestellt, eine Richtlehre 48 für den dorsalen Femurschnitt in eine Zylinderführung 49 der Schnittlehre 2 eingeschoben. Die Richtlehre 48 wird nun solange verschoben, bis sie in Kontakt zur distalen Femurfläche 50 besteht, wie in Fig. 3B dargestellt. Nun muß, wie in Fig. 3C gezeigt, durch Bewegen des Unterschenkels die Position der Richtlehre 48 so eingestellt werden, daß diese an der distalen Femurfläche 50 gleichmäßig anliegt.

Nach der Kontrolle der Spreizkraft kann die Richtlehre 48 mit einem Knochennagel 51, wie in Fig. 3D dargestellt, an der distalen Femurfläche 50 fixiert werden. Dadurch wird das System stabilisiert. Es muß dabei beachtet werden, daß die Richtlehre 48 immer noch gleichmäßig an der distalen Femurfläche 50 anliegt.

Danach wird durch die Sägeführung 37 der Schnittlehre 2 wie bei der distalen Femurosteotomie die Säge 47 eingeführt und die dorsale Femurosteotomie durchgeführt. Dabei muß auf den Schutz der ligamentären Strukturen geachtet werden.

Nach Ausführung der dorsalen Femurosteotomie wird der Knochennagel 51 und die Bänderspannvorrichtung 1 entfernt. Danach wird die Schnittlehre 2 von der Bänderspannvorrichtung 1 entfernt. Soweit nötig, werden nachbearbeitend dorsale Osteophyten entfernt.

Danach wird, wie in Fig. 3F dargestellt, die Bänderspannvorrichtung 1 erneut in den Gelenkspalt 43 eingebracht. Der Flexionsspalt wird anhand der Markierungen auf der Skala wie bereits in den Fig. 2A bis 2J beschrieben überprüft. Stimmt der gewünscht Flexionsspalt nicht mit dem gemessenen überein, muß an der dorsalen Femurfläche 52 nachreseziert werden (Wiederholung der in Fig. 3A bis 3F dargestellten Schritte).

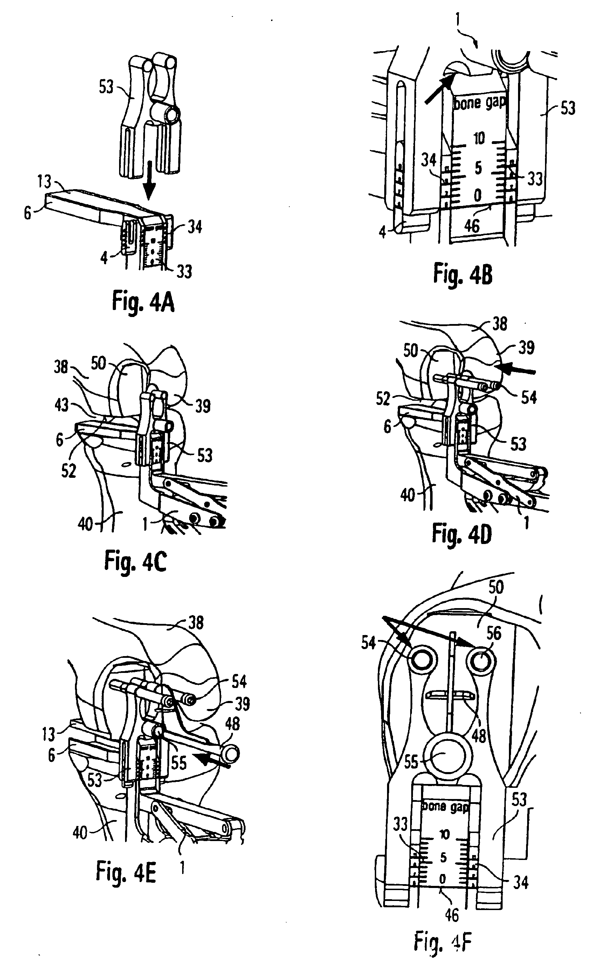

Die Fig. 4A bis 4J stellen die Arbeitsschritte für die abschließenden femuralen Schrägschnitte dar. Dabei wird zunächst, wie aus Fig. 4A ersichtlich, eine Bohrlehre 53 auf der Bänderspannvorrichtung 1 montiert, welche zum Bohren von Löchern für den Ansatz eines Schrägschnittblocks benötigt wird. Die Bohrlehre 53 wird dabei in die Halterungen 4 eingeschoben, welche auch für die Schnittlehre 2 verwendet werden. Die Bohrlehre 53 muß dabei so montiert werden, daß sie bis zum Anschlag auf der Bänderspannvorrichtung 1 aufgesetzt wird, wie aus Fig. 4B ersichtlich.

Danach wird die Bänderspannvorrichtung 1 wie bei den vorigen Schritten in den Kniegelenksspalt 43 eingebracht, wie aus Fig. 4C ersichtlich. Danach werden zwei Bohrhülsen 54 durch die Bohrlehre 53 auf Anschlag an die distale Femurfläche 52 eingeschoben. Dies ist in Fig. 4D dargestellt. Weiterhin wird, wie aus Fig. 4E ersichtlich, die Bänderspannvorrichtung 1 mit der gewünschten Kraft aufgespreizt. Es wird nochmals nachkontrolliert, ob die Bohrlehre 53 auf der Bänderspannvorrichtung 1 flächig aufliegt. Nun wird die bereits bei der dorsalen Femurosteotomie verwendete Richtlehre 48 in die Zylinderführung 55 eingeschoben, wie in Fig. 4E dargestellt, und die Position der Richtlehre 48 wiederum so eingestellt, daß die Richtlehre 48 an der distalen Femurfläche 50 gleichmäßig anliegt. Bei genauer Einstellung des Spanners muß die Null-Markierung 46 mit der gewünschten Inlay-Dicke 34 übereinstimmen.

Nach einer weiteren Kontrolle der optimalen Spannung der Bänderspannvorrichtung 1 werden gemäß Fig. 4F zwei Löcher 56 mit einer Bohrtiefe von ca. 35 mm und einem Durchmesser von ca. 3,2 mm in die distale Femurfläche 50 gebohrt. Danach wird die Bänderspannvorrichtung 1 gelockert und aus dem Kniegelenksspalt 43 entfernt.

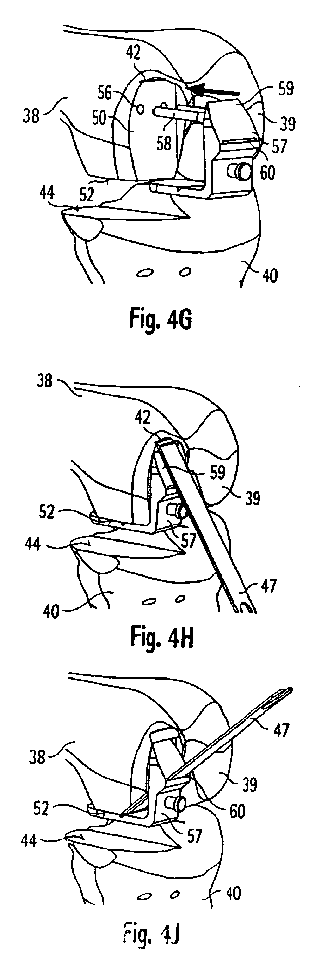

Fig. 4G zeigt das Einschieben der jeweils passend ausgewählten Schrägschnittlehre 57 mit zwei entsprechenden Stiften 58 in die beiden Löcher 56. Die Schrägschnittlehre 57 kann dabei eingeschoben oder auch mittels Haltezange geführt eingeschlagen werden. Die Schrägschnittlehre 57 ist so konzipiert, daß sie an der distalen Femurfläche 50 und der dorsalen Femurfläche 52 gerade anliegt.

Die Schrägschnittlehre 57 weist eine Anlagefläche 59 zur Ausführung des ersten, ventralen Schrägschnittes und eine Sägeführung 60 für den zweiten, dorsalen Schrägschnitt auf.

Fig. 4H zeigt dabei den ventralen Schrägschnitt, welcher bis zur ventralen Markierung 42, die zu Beginn der Operation mit dem Elektrokauter auf dem Femur 38 angebracht wurde, ausgeführt wird.

Fig. 4J zeigt den abschließenden dorsalen Schrägschnitt, wobei die Säge 47 durch die Sägeführung 60 geführt wird.

Nach Entfernen der Schrägschnittlehre 57 erfolgt eine Endbearbeitung der Tibia 40 und des Femur 38 und abschließend die Implantierung der femuralen und tibialen Implantate.

Die Erfindung ist nicht auf das dargestellte Ausführungsbeispiel beschränkt und läßt sich - wie bereits erwähnt - auch für beidseitige Implantate am Kniegelenk anwenden. Das Grundprinzip der Vorsehung von Halterungen für eine Schnittlehre an einer geeignet angepaßten Bänderspannvorrichtung läßt sich auch bei anderen Gelenken anwenden.

## Patentansprüche

1. Bänderspannvorrichtung (1) zur Vorbereitung für die Implantierung eines Gelenksimplantats mit einem Grundkörper (5), welcher eine erste Pratze (6) mit einer distalen Anlagefläche (7), welche auf einem ersten Knochen aufliegt, und eine zweite Pratze (13), die mit einer proximalen Auflagefläche (10) an einem zweiten Knochen anliegt, aufweist, wobei die zweite Pratze (13) parallel zur ersten Pratze (6) verschiebbar ist,
wobei eine Schnittlehre (2) auf Halterungen (4) des Grundkörpers (5) der Bänderspannvorrichtung (1) aufsetzbar ist und **dadurch gekennzeichnet,**
**daß** die Schnittlehre (2) Fortsätze (30) aufweist, welche U-förmig mit Schlitzen (31) ausgebildet sind.

2. Bänderspannvorrichtung nach Anspruch 1,
wobei
die Fortsätze (30) der Schnittlehre (2) mit den Halterungen (4) in Eingriff bringbar sind.

3. Bänderspannvorrichtung nach einem der Ansprüche 1 und 2,
wobei
die Schnittlehre (2) mittels eines Arretierungselements (3) an den Halterungen fixierbar ist.

4. Bänderspannvorrichtung nach einem der Ansprüche 1 bis 3,
wobei
die Halterungen (4) Rasten (32) umfassen.

5. Bänderspannvorrichtung nach Anspruch 4,
die Rasten (32) äquidistant sind.

6. Bänderspannvorrichtung nach Anspruch 4 oder 5,
wobei
die Schnittlehre (2) auf den Halterungen (4) rastend verschiebbar ist.

7. Bänderspannvorrichtung nach einem der Ansprüche 1 bis 6,
wobei
die erste Pratze (6) und die zweite Pratze (13) mittels einer Parallelverschiebevorrichtung (12) parallel zueinander verschiebbar sind.

8. Bänderspannvorrichtung nach Anspruch 7,
wobei an einem die zweite Pratze (13) mit der Parallelverschiebvorrichtung (12) verbindenden Bauteil (35) eine erste Skala (33) vorgesehen ist.

9. Bänderspannvorrichtung nach Anspruch 7,
wobei
an dem Grundkörper (5) eine zweite Skala (34) vorgesehen ist.

10. Bänderspannvorrichtung nach Anspruch 9,
wobei
die Skalen (33; 34) so zur Deckung bringbar sind, daß die Höhe eines in das zu behandelnde Gelenk einzusetzenden Implantats voreinstellbar ist.

11. Bänderspannvorrichtung nach einem der Ansprüche 1 bis 10,
wobei
die Schnittlehre (2) eine Zylinderführung (36) aufweist.

12. Bänderspannvorrichtung nach Anspruch 11,
wobei
in die Zylinderführung (36) eine Richtlehre (48) einführbar ist.

13. Bänderspannvorrichtung nach Anspruch 12, wobei
die Richtlehre (48) am zweiten Knochen mittels eines Knochennagels (51) fixierbar ist.

14. Bänderspannvorrichtung nach einem der Ansprüche 1 bis 13,
wobei
die Schnittlehre (2) eine Sägeführung (37) aufweist.

15. Bänderspannvorrichtung nach einem der Ansprüche 1 bis 14,
wobei
auf die Bänderspannvorrichtung (1) eine Bohrlehre (53) aufsteckbar ist.

16. Bänderspannvorrichtung nach Anspruch 15,
wobei
die Bohrlehre (53) auf die Halterungen (4) des Grundkörpers (5) aufsetzbar ist.

17. Bänderspannvorrichtung nach einem der Ansprüche 1 bis 16,
wobei
die Bänderspannvorrichtung (1) als zweiseitige Bänderspannvorrichtung (1) ausgeführt ist.

18. Bänderspannvorrichtung nach Anspruch 17,
wobei
die Bänderspannvorrichtung (1) eine Kraftanzeige (25) aufweist.

## Claims

1. Ligament-tensioning device (1) for preparing for the implantation of a joint implant, with a base body (5), having a first claw (6) with a distal bearing surface (7) which rests on a first bone, and a second claw (13) which rests, with a proximal bearing surface (10), against a second bone, the second claw (13) being displaceable parallel to the first claw (6),
wherein a cutting jig (2) can be placed onto mounts (4) of the base body (5) of the ligament-tensioning device (1), and
that the cutting jig (2) has projections (30) which are configured U-shaped with slots (31).

2. Ligament-tensioning device according to Claim 1, wherein the projections (30) of the cutting jig (2) can be brought into engagement with the mounts (4).

3. Ligament-tensioning device according to any of Claims 1 and 2, wherein the cutting jig (2) can be fixed to the mounts by means of a locking element (3).

4. Ligament-tensioning device according to any of Claims 1 to 3, wherein the mounts (4) comprise latches (32).

5. Ligament-tensioning device according to Claim 4, wherein the latches (32) are equidistant.

6. Ligament-tensioning device according to Claim 4 or 5, wherein the cutting jig (2) is displaceable on the mounts (4) in a latching manner.

7. Ligament-tensioning-device according to any of Claims 1 to 6, wherein the first claw (6) and the second claw (13) are displaceable parallel to one another by means of a parallel-displacement device (12).

8. Ligament-tensioning device according to Claim 7, wherein a first scale (33) is provided on a component (35) connecting the second claw (13) to the parallel-displacement device (12).

9. Ligament-tensioning device according to Claim 7, wherein a second scale (34) is provided on the base body (5).

10. Ligament-tensioning device according to Claim 9, wherein the scales (33; 34) can be brought into coincidence so that the height of an implant to be inserted into the joint to be treated can be preset.

11. Ligament-tensioning device according to any of Claims 1 to 10, wherein the cutting jig (2) has a cylindrical guide (36).

12. Ligament-tensioning device according to Claim 11, wherein an aligning jig (48) can be introduced into the cylindrical guide (36).

13. Ligament-tensioning device according to Claim 12, wherein the aligning jig (48) can be fixed to the second bone by means of a bone nail (51).

14. Ligament-tensioning device according to any of Claims 1 to 13, wherein the cutting jig (2) has a saw guide (37).

15. Ligament-tensioning device according to any of Claims 1 to 14, wherein a drilling jig (53) can be inserted onto the ligament-tensioning device (1).

16. Ligament-tensioning device according to Claim 15, wherein the drilling jig (53) can be placed onto the mounts (4) of the base body (5).

17. Ligament-tensioning device according to any of Claims 1 to 16, wherein the ligament-tensioning device (1) is configured as a bilateral ligament-tensioning device (1).

18. Ligament-tensioning device according to Claim 17, wherein the ligament-tensioning device (1) has a force indicator (25).

## Revendications

1. Dispositif de tensionnement de ligaments (1) destiné à la préparation pour l'implantation d'un implant articulé avec un corps de base (5) qui présente une première griffe (6) avec une surface d'appui distale (7) reposant sur un premier os, et une seconde griffe (13) reposant avec une surface d'appui proximale (10) sur un second os, la seconde griffe (13) étant coulissante parallèlement à la première griffe (6) ;
dans lequel un modèle de coupe (2) pouvant être appliqué sur des supports (4) du corps de base (5) du dispositif de tensionnement de ligaments (1),
**caractérisé en ce que** le modèle de coupe (2) présente des prolongements (30) qui ont la forme d'un U avec des fentes (31).

2. Dispositif de tensionnement de ligaments selon la revendication 1, dans lequel
les prolongements (30) du modèle de coupe (2) peuvent être emboîtés dans les supports (4).

3. Dispositif de tensionnement de ligaments selon l'une quelconque des revendications 1 et 2, dans lequel
le modèle de coupe (2) peut être fixé au moyen d'un élément de blocage (3) sur les supports.

4. Dispositif de tensionnement de ligaments selon l'une quelconque des revendications 1 à 3, dans lequel
les supports (4) comprennent des crans (32).

5. Dispositif de tensionnement de ligaments selon la revendication 4, dans lequel
les crans (32) sont équidistants.

6. Dispositif de tensionnement de ligaments selon la revendication 4 ou 5, dans lequel
le modèle de coupe (2) est coulissant par crans sur les supports (4).

7. Dispositif de tensionnement de ligaments selon l'une quelconque des revendications 1 à 6, dans lequel
la première griffe (6) et la seconde griffe (13) sont coulissantes parallèlement l'une à l'autre au moyen d'un dispositif de déplacement parallèle (12).

8. Dispositif de tensionnement de ligaments selon la revendication 7, dans lequel
une première graduation (33) est prévue sur un composant (35) reliant la seconde griffe (13) au dispositif de déplacement parallèle (12).

9. Dispositif de tensionnement de ligaments selon la revendication 7, dans lequel
une seconde graduation (34) est prévue sur le corps de base (5).

10. Dispositif de tensionnement de ligaments selon la revendication 9, dans lequel
les graduations (33 ; 34) peuvent être placées en recouvrement, de telle sorte que la hauteur d'un implant à mettre en oeuvre dans l'articulation à traiter est préréglable.

11. Dispositif de tensionnement de ligaments selon l'une quelconque des revendications 1 à 10, dans lequel
le modèle de coupe (2) présente un guidage cylindrique (36).

12. Dispositif de tensionnement de ligaments selon la revendication 11, dans lequel
un modèle dirigé (48) peut être introduit dans le guidage cylindrique (36).

13. Dispositif de tensionnement de ligaments selon la revendication 12, dans lequel
le modèle dirigé (48) peut être fixé sur le second os au moyen d'un clou à os (51).

14. Dispositif de tensionnement de ligaments selon l'une quelconque des revendications 1 à 13, dans lequel
le modèle dirigé (2) présente un guidage de scie (37).

15. Dispositif de tensionnement de ligaments selon l'une quelconque des revendications 1 à 14, dans lequel
un calibre de perçage (53) peut être emboîté sur le dispositif de tensionnement de ligaments (1).

16. Dispositif de tensionnement de ligaments selon la revendication 15, dans lequel
le calibre de perçage (53) peut être posé sur les supports (4) du corps de base (5).

17. Dispositif de tensionnement de ligaments selon l'une quelconque des revendications 1 à 16, dans lequel
le dispositif de tensionnement de ligaments (1) est conçu comme un dispositif de tensionnement de ligaments (1) à deux faces.

18. Dispositif de tensionnement de ligaments selon la revendication 17, dans lequel
le dispositif de tensionnement de ligaments (1) présente un affichage de la force (25).
